(19) Europäisches Patentamt
European Patent Office
Office européen des brevets

(11) Publication number: **0 300 812 A2**

(12) **EUROPEAN PATENT APPLICATION**

(21) Application number: **88306758.9**

(22) Date of filing: **22.07.88**

(51) Int. Cl.[4]: **C 07 F 15/00**
A 61 K 31/28

(30) Priority: **24.07.87 US 77269**

(43) Date of publication of application:
**25.01.89 Bulletin 89/04**

(84) Designated Contracting States:
**AT BE CH DE ES FR GB GR IT LI LU NL SE**

(71) Applicant: **ENGELHARD CORPORATION**
**Menlo Park CN 28**
**Edison New Jersey 08818 (US)**

(72) Inventor: **Amundsen, Alan R.**
**12 Meadowbrook Drive**
**Somerville New Jersey 08876 (US)**

**Stern, Eric W.**
**234 Oak Tree Road**
**Mountainside New Jersey 07092 (US)**

**Hollis, Leslie Steven**
**33 Guilford Lane**
**Mercerville New Jersey 08619 (US)**

(74) Representative: **Fisher, Adrian John et al**
**CARPMAELS & RANSFORD 43 Bloomsbury Square**
**London WC1A 2RA (GB)**

(54) **Platinum triamine antitumor agents containing saturated amines.**

(57) Novel platinum triamine antitumor agents containing saturated amines are disclosed. These complexes exhibit an antitumor effect and are characterised by low mammalian toxicity.

EP 0 300 812 A2

## Description

## PLATINUM TRIAMINE ANTITUMOR AGENTS CONTAINING SATURATED AMINES

### Field of the Invention

The present invention relates to novel platinum triamine complexes, to pharmaceutical compositions containing such a complex in admixture with a pharmaceutically acceptable diluent or carrier, and to the use of such complexes in therapy, in particular for the treatment of malignant tumors in animals, especially in mammals.

### Background of the Invention

Cisplatin has been demonstrated to be effective in the treatment of a variety of forms of cancer such as testicular and ovarian carcinoma. A continuing effort has been made in analog development programs to broaden the spectrum of activity and to improve the therapeutic properties of platinum-based antitumor agents. These goals include developing agents that demonstrate activity in unresponsive diseases such as colon, breast, stomach and brain cancer, and improving on the activity seen in tumor systems that presently respond to cisplatin such as lung, bladder and head and neck carcinomas. The research in this area has produced a number of promising new compounds that have shown good activity in various animal tumor screens. See, for example, Prestayko, A.E. et al, "Cisplatin, Status and New Developments" Academic Press: New York 1980 and Hacker, M.P. et al, "Platinum Coordination Complexes in Cancer Chemotherapy"; Martinus-Nijhoff: Boston 1984.

A number of these compounds are being evaluated in the clinic with the hope of developing an effective platinum based antitumor agent that has a significantly different spectrum of activity and improved toxicology properties. Also important are compounds with superior physical and chemical properties such as aqueous solubility and stability greater than cisplatin.

During the past fifteen (15) years, several thousand platinum complexes have been prepared and evaluated in a variety of animal tumor screens in an attempt to devise structure-activity relationships among the platinum analogs. The classical structure-activity relationships were first summarized by Cleare and Hoeschele in 1974 and since that time these guidelines have continued to dominate the search for new analogs. In general, platinum(II) complexes of the form cis-$PtA_2X_2$, wherein A is an amine ligand and X is an anionic leaving group, represent the majority of the active analogs. The corresponding trans-diamine complexes are devoid of activity. The importance of the cis geometry of the leaving groups has been stressed in many studies that suggest the mechanism of antitumor activity of these compounds is related to their ability to inhibit replication by binding two adjacent guanine bases on duplex DNA. See, for example, Pinto et al, Biochem. Biophys. Acta 1985, 780, 167, Johnson et al, "Biochemical Mechanisms of Platinum Antitumor Drugs", IRL Press, Washington, D.C., 1986, page 1 and Roberts et al, "Biochemical Mechanisms of Platinum Antitumor Drugs", page 29.

Aside from those complexes conforming to the basic cis-$PtA_2X_2$ structural class, relatively few platinum compounds have demonstrated activity in in vivo tumor systems. U.S. patent application Serial No. 723,783 filed on April 16, 1985 naming the same inventors as named herein (and corresponding to EP-A-O,199,524) discloses a series of platinum antitumor agents which violate the classical structure-activity relationships. The compounds disclosed therein are triamine complexes represented by the following general formula:

(I)

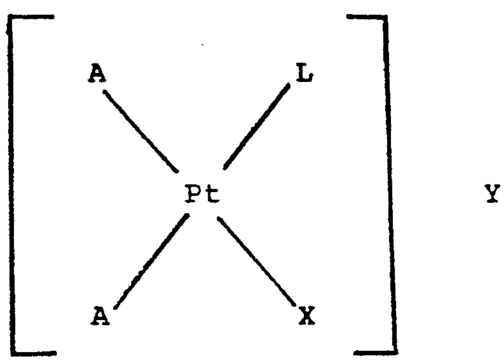

wherein A is a monodentate amine, such as $NH_3$ or $RNH_2$ (aliphatic amine), or a bidentate ($A_2$) ligand such as

ethylenediamine, L is a heterocyclic unsaturated amine such as pyridine, X is a monodentate anionic ligand, and Y is a monovalent anion.

In addition to the disclosure in that U.S. Patent application some other triamine compounds have been reported in the literature. These include cis-[Pt($NH_3$)$_2$(2-hydroxypyridine)Cl]Y (Y=Cl, $NO_3$) (L.S. Hollis, S.J. Lippard, Inorg. Chem. 1983, 22, 2708), cis-[Pt($NH_3$)$_2$(1-methylcytosine)Cl]X (X=Cl, $NO_3$) (B. Lippert, C.J.L. Lock, R.A. Speranzini, Inorg. Chem. 1981, 20, 335, J.F. Britten, B. Lippert, C.J.L. Lock, P. Pilon, Inorg. Chem. 1982, 21, 1936), [Pt(en)(py)Cl]Cl (en=ethylenediamine, py=pyridine) (Y.N. Kukuskin, V.P. Kotelnikov, V.N. Spevak, Zh. Obshch.Khim. 1973, 43, 2352), and cis-[Pt($NH_2OH$)$_2$(3-pyridinecarboxamide)Cl]Cl (L.S. Tikhonova, A.I. Stetsenko, Koord. Khim. 1983, 9, 2660). In all of the above cases no antitumor activity was disclosed.

Two other compounds, cis-[Pt($NH_3$)$_2$(diethylamine)Cl]Cl and cis-[Pt($NH_3$)$_2$(triethylamine)Cl]Cl have been prepared (Zheligovskaya et al, Koord. Khim. 1979, 5, 1527 and Zheligovskaya et al, Zh. Anal. Khim. 1982, 37, 72). Another related species [Pt($NH_3$)$_2$($HOCH_2$)$_3$C$NH_2$)Cl]Cl appears to be a trans compound. (Stetsenko et al, Zh. Neorg. Khim 1981, 26, 3145).

Three cis-[Pt($NH_3$)$_2$LX]X compounds have been prepared in which the L ligands are the amino acids glycine, 4-amino butyric acid and proline. See Picova et al, J. Inorg. Biochem. 1985, 23, 43, Appelton, Aust. J. Chem. 1986, 39, 1347, Bolshtein et al, Zh. Neorg. Khim. 1980, 25, 231, respectively. The X groups in these compounds are Cl and Br.

[$PtL_3X$]X species have also been cited in the literature. The L ligands mentioned include n-propylamine, n-hexylamine, n-dodecylamine and piperdine. See Cherchi et al, Transition Met. Chem. 1985, 10, 26, Faraglia et al, Thermochim. Acta 1984, 78 159, Pregosin et al, J. Amer. Chem. Soc. 1973, 95, 2047, and Birnbaum et al, J. Inorg. Nucl. Chem. 1973, 35, 3145, respectfully. In these compounds X is Cl or Br.

## Summary of the Invention

The platinum compounds of this invention are a new series of platinum triamine antitumor agents containing saturated amines. These compounds exhibit excellent activity against malignant tumors in animals which are sensitive thereto and have low mammalian toxicity. In addition, the compounds have good solubility and stability in water.

The present invention is directed to compounds of the following general formula:

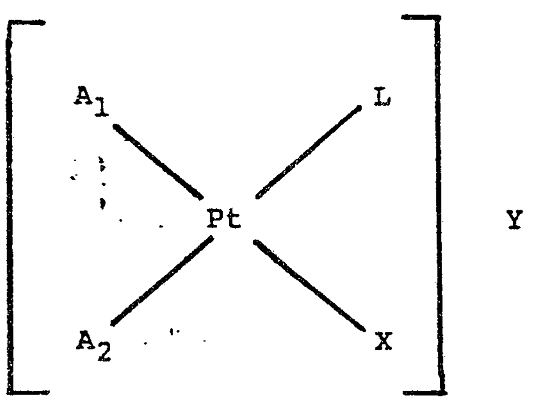

wherein $A_1$ and $A_2$ are the same or different and are a monodentate amine, such as $NH_3$ or $R^1NH_2$, wherein $R^1$ is a linear or branched $C_1$-$C_6$ alkyl group, which may be substituted with one or more substituents such as hydroxy, $C_1$-$C_4$ alkoxy, halo, carboxy, $C_1$-$C_4$ alkylcarboxy, $C_1$-$C_4$ alkoxycarbonyl or $C_3$-$C_6$ cycloalkyl. $A_1$ and $A_2$ taken together represent a diamine of the general formula

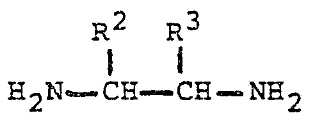

wherein $R^2$ and $R^3$, taken separately are defined as $R^1$ above. $R^2$ and $R^3$ taken together form a 1,2-diaminocycloalkane containing 4-8 nuclear carbon atoms which may be substituted on the carbons by one or more substituents such as hydroxy, $C_1$-$C_4$ alkoxy, halo, carboxy, $C_1$-$C_4$ alkylcarboxy, $C_1$-$C_4$ alkoxycarbonyl,

or $C_3$-$C_6$ cycloalkyl.

L is different from $A_1$ and $A_2$ and is a $C_1$-$C_{12}$ branched, $C_3$-$C_{12}$ cyclic, $C_5$-$C_{12}$ bicyclic, $C_2$-$C_{12}$ heterocyclic or $C_5$-$C_{12}$ bicyclic heterocyclic, saturated amine which may be substituted by one or more substituents such as hydroxy, $C_1$-$C_4$ alkoxy, halo, $C_1$-$C_4$ alkylcarboxy, $C_1$-$C_4$ alkoxycarbonyl, or $C_3$-$C_6$ cycloalkyl and in the case of the cyclic, bicyclic, heterocyclic and bicyclic heterocyclic saturated amines the substituent $C_1$-$C_4$ alkyl. L is also a substituted linear $C_1$-$C_{12}$ amine containing as substituents one or more of the substituents recited in this paragraph. L may also be a linear unsubstituted $C_1$-$C_{12}$ amine, although the only linear unsubstituted amine tested as of the date of this application, octylamine, showed substantially no activity in the S-180 ascites screen. The octylamine and/or other $C_1$-$C_{12}$ linear amines may exhibit activity in other cancer screens such as L1210, P388, B16 melanoma, Lewis Lung carcinoma, M5076, human tumor xenografts and in vitro human tumor cell lines. In addition, other linear amines may be tested in the S-180 ascites screen for activity.

X is an anion such as halo, hydroxo, pseudohalo, $HSO_4$, $H_2PO_4$, ascorbate, $C_1$-$C_6$ carboxylate.

Y is an anion such as the anions described herein for X and in addition nitrate or perchlorate. X may or may not be the same as Y.

## Description of preferred embodiments

## Pharmacology

The products of this invention are useful in the treatment of malignant tumor cells sensitive thereto in animals as, for example, Sarcoma 180 ascites and L1210 leukemia in mammals such as mice. This antitumor cell effect also may extend to other sarcomas and leukemias and to such other tumor cells as lymphoid leukemia, lymphosarcoma, myelocytic leukemia, malignant lymphoma, squamous cell carcinoma, adenocarcinoma, scirrhous carcinoma, malignant melanoma, seminoma, teratoma, choriocarcinoma, embryonal carcinoma, cystadenocarcinoma, endometroidcarcinoma or neuroblastoma and the like. In addition, the complexes may be useful as anti-viral, anti-inflammatory, anti-bacterial and anti-parasitic agents.

The complexes of this invention may be administered parenterally or orally in a mixture with a non-toxic pharmacologically acceptable inert carrier or diluent in any of the usual pharmaceutical forms. These include solid and liquid oral unit dosage forms such as tablets, capsules, powders and suspensions or solutions and suspensions for subcutaneous, intramuscular, intravenous, or intraarterial injection.

The term "unit dosage" refers to physically discrete units which may be administered in single or multiple dosages each containing a predetermined quantity of active ingredient in association with the required diluent, carrier or vehicle. The quantity of active ingredient is the amount of the complex which is needed to produce the desired therapeutic effect.

A typical unit dosage consists essentially of from about to 10-450 mg of active ingredient; however, the form in which the ingredient is administered and the frequency of administration is usually determinative of the concentration. Thus, for example, oral unit dosage forms containing 20 to 450 mg of active ingredient may be administered one or more times per day depending upon the severity of the tumor cells which is sought to be treated and the condition of the host animal. By contrast, parenteral administration generally requires from about 10 to about 100 mg of the active ingredient per unit dosage administered as a daily dose or as a fraction thereof depending upon whether the regimen calls for administration once, twice, three or four times daily.

By contrast to the unit dosage, the effective dose is that dosage which is needed to achieve the desired anti-tumor effect. In general, this dosage lies within the range of from about 10 to 950 mg of the active ingredient per kg of body weight of the host animal. A preferred concentration lies within the range of from about 30 to 450 mg/kg of body weight. For oral administration it has been found that an effective dose of about 50 to 950 mg/kg is most suitable, whereas in the case of parenteral administration it is usually advisable to employ from about 30 to 350 mg/kg. These unit dosages are well below the toxic or lethal dose and they may be varied over a wide range for adjustment to the patient who is being treated.

According to the present invention, the term "pharmacologically acceptable inert carrier or diluent" means a non-toxic substance which, when mixed with the active ingredient, renders it more suitable for administration. Compositions intended for oral administration may include such carriers or diluents as corn starch, potato starch, sodium carboxymethyl cellulose, ethyl cellulose, cellulose acetate, powder gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and dicalciumphosphate. The compositions may also contain non-toxic adjuvants and modifiers such as dyes, buffering agents, preservatives, surfactants, emulsifiers, flavoring agents, biocides and the like.

Tablets are prepared by mixing a complex of this invention in a suitably comminuted or powdered form with a diluent or base such as starch, kaolin, dicalciumphosphate and the like. The resulting mixture can be granulated by wetting with a binder such as a syrup, starch (paste), acacia mucilage or solutions of cellulosic or polymeric materials, whereafter, the wetted mixture is formed through a screen. As an alternative to granulating, the powder mixture can be run through a tablet machine and any imperfectly formed slugs broken into granules. The granules are lubricated to prevent sticking to the tablet-forming dyes via the addition of

stearic acid, a stearate salt, talc or mineral oil and the lubricated mixture is then compressed into tablets. The complexes can also be combined with free flowing inert carriers followed by compression into tablets without going through the granulated or slugging steps. A protective coating or sealing coat of shellac, sugar or polymeric material and a polished coating of wax can also be provided. Dye stuffs may be added to distinguish different unit dosages.

Dry or hard filled capsules are formulated by preparing a powdered mixture, according to the procedure herein before described and pouring the mixture into preformed gelatin sheets. A lubricant such as talc, magnesium stearate or calcium stearate can be added prior to the filling operation. A glidant such as coloidal silica may be added to improve the flow characteristics and a disintegrating or solubilizing agent may also be added to enhance the effectiveness of the medicament upon ingestion.

In soft gelatin capsules, the active complex is dissolved or suspended in vegetable oil, peanut oil, alcohol or glycerine and the like.

Powders for addition to foods, drinking water, fruit juice or other potable liquids are prepared by comminuting the compound to a fine size and mixing with a similarly comminuted pharmaceutical diluent or carrier such as an edible carbohydrate as, for example, starch. Sweetening agents and flavorings, preservatives and dispersing and/or coloring agents may also be employed.

Oral fluids such as syrups and elixirs are prepared in unit dosage forms so that a given quantity of medicament, such as a teaspoonful, will contain a predetermined amount of the active ingredient. Suspensions can be formulated by dispersing the active ingredient in a non-toxic vehicle in which it is essentially insoluble.

Compositions intended for parenteral administration may include such diluents and carriers as water or water-miscible solvents as, for example, sesame oil, groundnut oil, aqueous propylene glycol and a solution of sodium riboflavin. Typical of said compositions are solutions which contain the active ingredient in sterile form. Alternatively, a unit dosage form for parenteral administration can be prepared by placing a measured amount of complex in solution in sterile form, removing the solvent by lyophilization and sealing the vial. An accompanying vial of sterile vehicle can be provided for mixing with the complex prior to administration.

According to the present invention one or more of the complexes may be combined into a single unit dosage form or, alternatively, one or more of the complexes of the present invention may be combined with other known anti-tumor agents, therapeutic agents or nutrative agents so as to enhance or compliment the antitumor effect.

The preferred compositions for oral administration are tablets in which the complexes of the present invention are present in quantities of about 5 to about 375 mg but, preferably, about 10 to 200 mg in a pharmaceutically acceptable orally ingestible solid carrier. If desired, the compositions may also contain flavorants, binders, lubricants and other excipients known in the art.

A preferred alternative for oral administration is the soft gelatin capsule. Such a composition may contain from about 5 to about 375 mg, but, preferably, about 10 to 200 mg by weight of active ingredient dissolved or suspended in vegetable oil, peanut oil, alcohol or glycerin and the like.

A preferred unit dosage form for parenteral administration will contain complexes of the present invention of about 10 to 100 mg, but preferably 15 to 75 mg.

The following embodiments illustrate representative unit dosage forms:

## Compressed Tablet

| | |
|---|---|
| cis-[Pt$(NH_3)_2$(4-methylpiperidine)Cl]$NO_3$ | 200 mg. |
| Niacinamide | 50 mg. |
| Calcium Pantothenate | 20 mg. |
| Magnesium Sulfate | 50 mg. |
| Zinc Sulfate | 50 mg. |
| Magnesium Stearate | 10 mg. |
| | 380 mg. |

The cis-[Pt$(NH_3)_2$(4-methylpiperidine)Cl]$NO_3$ complex, niacinamide, calcium pantothenate, magnesium sulfate, zinc sulfate and magnesium stearate (5.0 mg.) are mixed and compressed into slugs. The slugs are then broken into granules and sifted through an 8 mesh screen. Additional magnesium stearate (5.0 mg.) is added and the mixture is then compressed into tablets suitable for oral administration.

## Soft Gelatin Capsule

A soft elastic gelatin capsule is filled with the following ingredients:

| | |
|---|---|
| cis-[Pt($NH_3$)$_2$(cyclohexanemethylamine)Cl]$NO_3$ | 200 mg. |
| Wheat germ oil | 50 mg. |
| Sunflower seed oil | 100 mg. |
| | 350 mg. |

The cis-[Pt($NH_3$)$_2$(cyclohexanemethylamine)Cl]$NO_3$ complex and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

## Dry Filled Capsule

A hard dry-filled capsule may be prepared from the following ingredients:

| | |
|---|---|
| cis-[Pt($NH_3$)$_2$(4-methylpiperidine)Cl]$NO_3$ | 250 mg. |
| Niacinamide | 50 mg. |
| Calcium Pantothenate | 10 mg. |
| Sodium Ascorbate | 150 mg. |
| | 460 mg. |

The cis-[Pt($NH_3$)$_2$(4-methylpiperidine)Cl]$NO_3$ complex is reduced to a No. 60 powder. Niacinamide, calcium pantothenate and sodium ascorbate are passed through a No. 60 bolting cloth and these ingredients are added to the platinum diamine-4-methylpiperidine complex. This combination of ingredients is mixed for 10 minutes and then poured into a No. 3 size gelatin capsule.

## Dry Powder

The following composition illustrates a representative dosage in dry powder form. In this embodiment the active ingredient is water soluble and it is combined with up to 60% by weight of a suitable flavoring agent. All quantities are in a weight-percent relationship.
cis-[Pt($NH_3$)$_2$(cyclohexanemethylamine)Cl]$NO_3$ 25-90%
Flavoring Agent 10-60%
Preservative 0.1%

## Parenteral Solution

Injectable solutions can be formulated by mixing an ampoule of active ingredient with an ampoule of sterile diluent:
cis-[Pt($NH_3$)$_2$(4-methylpiperidine)Cl]$NO_3$ 100 mg.
Ampoule: Sterile water (Diluent for Injection) 5 cc.

The cis-[Pt($NH_3$)$_2$(4-methylpiperidine)Cl]$NO_3$ complex and water are mixed thoroughly immediately prior to administration. If desired, one or more other active ingredients may be added to provide an injectable solution having enhanced therapeutic activity.

## Preparative Methods

The compounds of this invention may be prepared as follows: cis-$PtA_2Cl_2$ is reacted with an equimolar amount of $AgNO_3$ in DMF for 6-72 hr (22-48 hr preferred) at 0-50 °C (20-30 °C preferred). The AgCl precipitate is removed by filtration and an equimolar amount of L added to the filtrate. This is reacted for 8-48 hr (16-24 hr preferred) at 0-50 °C (20-30 °C preferred). The solvent is then removed under vacuum. The materials are

purified by shaking the residue with $CH_2Cl_2$ to remove excess L and DMF, and recrystallization from hot water. Contamination with cis-$PtA_2Cl_2$ can be removed by recrystallization of the crude product from hot methanol or by first dissolving the product in a minimum of water (at 20-30 °C) and gradually adding absolute ethanol. The cis-$PtA_2Cl_2$ impurity (yellow) is precipitated first and removed by filtration. Further addition of ethanol followed by removal of ethanol/water azeotrope under vacuum produces a precipitate of purified product.

Examples 1-7 illustrate the methods by which compounds of disclosed herein may be prepared. Example 8 describes the protocol used to evaluate their efficacy in mice. These examples are illustrative only and the invention should not be construed as being limited thereto because it will be apparent to one of ordinary skill that obvious modifications may be effected and functionally equivalent reagents may be substituted for those recited without departing from the spirit or scope of this invention.

The compounds of the Examples were characterized by $^{195}$Pt NMR (Table 1), $^{13}$C NMR (Table 2) and by elemental analysis. HPLC was used to determine that the products were free of contamination by cis-$PtA_2Cl_2$, tetramine (cis-$[PtA_2L_2]X_2$) and free Ligand L.

## Example 1

### cis-$[Pt(NH_3)_2Cl_2]$

In the present invention, the cis-$[Pt(NH_3)_2Cl_2]$ starting material was prepared from $K_2[PtCl_4]$ using the method of Dhara as described in Dhara, S.G.; Indian J. Chem. 1970, 8, 193 which is incorporated herein by reference.

## Example 2

### cis-$[Pt(NH_3)_2$(4-methylpiperidine)Cl]$NO_3$

Cisplatin (6.0 g) and $AgNO_3$ (3.39 g) were stirred in 100 mL DMF for 48 hr at room temperature. After filtering, 4-methylpiperidine (2.28 mL) was added and the solution stirred for 72 hr at room temperature. It was then filtered and taken to dryness under vacuum. The residue was stirred with $CH_2Cl_2$ for 4 hr. A yellow solid was filtered, and recrystallized from 30 mL hot water. The resulting crystals were treated with ca 100 mL hot methanol. The methanol solution was filtered while hot, and the methanol evaporated, leaving a white solid (yield 2.25 g). Anal. Calc. for $PtC_6H_{17}N_4O_3Cl$: C, 17.01; H, 4.04; N, 13.22. Found: C, 16.89; H, 4.63; N, 13.29.

## Example 3

### cis-$[Pt(NH_3)_2$(cyclohexanemethylamine)Cl]$NO_3$

Cisplatin (6.0 g) and $AgNO_3$ (3.39 g) were stirred in 100 mL DMF for 24 hr at room temperature. The solution was filtered to remove AgCl, and cyclohexanemethylamine (2.60 mL) added, followed by additional stirring for 24 hr at room temperature. The resulting solution was taken to dryness under vacuum, the remaining yellow solid was shaken for 2 hr with 100 mL $CH_2Cl_2$, and the solid collected on a filter. This material was extracted with 200 mL hot methanol, the methanol removed under vacuum and the residue recrystallized from hot water, then from hot methanol. Two identical crops of product resulted; the total yield was 0.82 g. Anal. Calc. for $PtC_7H_{21}N_4O_3Cl$: C, 19.12; H, 4.81; N, 12.74. Found: C, 19.38; H, 4.82; N, 12.72.

## Example 4

cis-[$Pt(NH_3)_2$(isopropylamine)Cl]$NO_3$

Cisplatin (6.0 g) and $AgNO_3$ (3.39 g) were stirred in 100 mL DMF for 24 hr at room temperature. After filtration of the resulting AgCl, isopropylamine (1.7 mL) was added and the solution stirred for an additional 24 hr. The DMF was then removed under vacuum and the residue shaken with 150 mL $CH_2Cl_2$ for 1.5 hr. A yellow solid was collected; this was dissolved in a small volume (10 mL) of water and filtered to remove the bulk of the cisplatin present. At this point 150 mL ethanol was added and evaporation of ethanol/water azeotrope begun under vacuum. At a volume of ca. 20 mL, a white solid began to form, and the mixture was refrigerated overnight. The solid was collected and recrystallized twice from hot water yielding 1.67 g white solid. Anal. Calc. for $PtC_3H_{15}N_4O_3Cl$: C, 9.34; H, 3.92; N, 14.53. Found: C, 9.35; H, 3.90; N, 14.53.

Example 5

cis-[$Pt(NH_3)_2$(quinuclidine)Cl]$NO_3$

Cisplatin (6.0 g) and $AgNO_3$ (3.39 g) were stirred in 100 mL DMF for 72 hr at room temperature. The resulting AgCl precipitate was removed by filtration and quinuclidine (2.22 g) added. The reaction mixture was stirred at room temperature for 24 hr. The DMF was removed under vacuum and the residue shaken with 100 mL $CH_2Cl_2$ for 2 hr. The crude product was filtered and extracted with 200 mL hot methanol. Upon cooling a solid crystallized and was removed by filtration. A second crop of solid was obtained by reducing the volume of the solution to ca. 20 mL and refrigerating for 2-3 hr. The two crops were combined and treated a second time with 100 mL hot methanol, obtaining two crops as above. The second of these was dissolved in 20 mL hot water. Ethanol (200 mL) was added and the volume of solution reduced under vacuum until crystals began to appear and the solution refrigerated for 24 hr. A white crystalline solid was collected, washed with ethanol and vacuum dried. The yield 0.46 g.

Example 6

cis-[$Pt(NH_3)_2$(2-amino-1-methoxypropane)Cl]$NO_3$

Cisplatin (6.0 g) and $AgNO_3$ (3.39 g) were stirred in 100 mL DMF for 24 hr. After removing the AgCl by filtration, 2-amino-1-methoxypropane (2.1 mL) was added to the filtrate and stirring continued for another 24 hr. The DMF was then removed under vacuum and the residue shaken with $CH_2Cl_2$ for 2 hr. The crude product was filtered and dissolved in 10-15 mL water. After filtration of insolubles, 150 mL ethanol was added and reduction of volume under vacuum begun. As the volume was reduced, small amounts of yellow solid appeared and were removed by filtration. when a white solid began to crystallize the solution was refrigerated overnight. The solid was collected and recrystallized twice from hot methanol using decolorizing charcoal. The white solid product was washed with methanol and vacuum dried. The yield of white needles was 0.98 g. Anal. Calc. for $PtC_4H_{17}N_4O_4Cl$: C, 11.56; H, 4.12; N, 13.48. Found: C, 11.43; H, 4.08; N, 13.31.

EXAMPLE 7

cis-[$Pt(NH_3)_2$(octylamine)Cl]$NO_3$

Cisplatin (6.0 g) and $AgNO_3$ (3.39 g) were stirred in 100 mL DMF overnight at room temperature. After filtration, 1-octylamine (3.3 mL) was added and the reaction mixture stirred for an additional 24 hr. The DMF was removed under vacuum and the residue shaken with 150 mL $CH_2Cl_2$ for 3 hr. The resulting solid was extracted with ca 100 mL hot methanol. On evaporation of the methanol filtrate under vacuum a

cisplatin-containing precipitate formed initially and was removed by filtration. Further concentration to ca 30 mL and refrigeration overnight yielded 2.36 g product. Anal. Calc. for $PtC_8H_{25}N_4O_3Cl$: C, 21.08; H, 5.53; N, 12.29; Found: C, 21.02; H, 5.55; N, 12.25.

Table 1

$^{195}$Pt NMR Data for Pt Triamines Containing Saturated Amines

| Compound | Chemical Shift[a] |
|---|---|
| *cis*-[Pt($NH_3$)$_2$(4-methylpiperidine)Cl]$NO_3$ | -2411 |
| *cis*-[Pt($NH_3$)$_2$(cyclohexanemethylamine)Cl]$NO_3$ | -2408 |
| *cis*-[Pt($NH_3$)$_2$(isopropylamine)Cl]$NO_3$ | -2410 |
| *cis*-[Pt($NH_3$)$_2$(octylamine)Cl]$NO_3$ | -2406 |
| *cis*-[Pt($NH_3$)$_2$(quinuclidine)Cl]$NO_3$ | -2304 |
| *cis*-[Pt($NH_3$)$_2$(2-amino-1-methoxypropane)Cl]$NO_3$ | -2407 |

[a] in ppm vs. $H_2PtCl_6$ at 0 ppm.

Table 2

$^{13}C$ NMR Data for Pt Triamines Containing Saturated Amines

| Compound | Chem. Shift | Assignment |
| --- | --- | --- |
| *cis*-[Pt($NH_3$)$_2$(4-methylpiperidine)Cl]$NO_3$ | 52.6 | C2,C6 |
| | 34.4 | C3,C5 |
| | 29.6 | C4 |
| | 21.2 | C7 |
| *cis*-[Pt($NH_3$)$_2$(cyclohexanemethylamine)Cl]$NO_3$ | 52.9 | C1 |
| | 38.7 | C2 |
| | 30.4 | C3,C7 |
| | 25.7 | C4,C6 |
| | 26.3 | C5 |
| *cis*-[Pt($NH_3$)$_2$(isopropylamine)Cl]$NO_3$ | 23.0 | C1,C3 |
| | 48.6 | C2 |
| *cis*-[Pt($NH_3$)$_2$(octylamine)Cl]$NO_3$ | 46.4 | C1 |
| | 31.1 | C2 |
| | 30.3 | C3 |
| | 28.4,28.4 | C4,C5 |
| | 25.8 | C6 |
| | 22.1 | C7 |
| | 13.5 | C8 |
| *cis*-[Pt($NH_3$)$_2$(quinuclidine)Cl]$NO_3$ | 54.5 | C2,C6,C7 |
| | 25.7 | C3,C5,C8 |
| | 19.1 | C4 |
| *cis*-[Pt($NH_3$)$_2$(2-amino-1-methoxy-propane)Cl]$NO_3$ | 75.5 | C1 |
| | 51.5 | C2 |
| | 17.3 | C3 |
| | 58.4 | C(MeO) |

[a] in ppm vs. TMS at 0 ppm.

## Example 8

## Antitumor Evaluation

The platinum triamines of Examples 2-7 were tested for antitumor activity using both the Sarcoma 180 ascites and the L1210 Leukemia tumor models. Each compound was evaluated in at least one of these screens.

## L1210 Screening Methodology

L1210 screening was conducted according to a published method. See Rose, W.C.; Schurig, J.F.; Huftalen, J.B.; Bradner, W.T. Cancer Treat. Rep. 1982, 66, 135, which is incorporated herein by reference. Female $CDF_1$ mice (16 - 22 g) were implanted with 1 x $10^6$ tumor cells ip on day zero. Compounds were administered in 0.5 mL water ip on day one. Groups of 6 mice were used for each dose. A control group received only tumor and 0.5 mL water. A positive control group received tumor and 8 mg/kg cisplatin in 0.5 mL of 0.15 M NaCl. The test was terminated after three times the mean survival time (MST) of the control group, surviving mice were counted as dying on that day. Activity was determined based on the percent increase in MST of test mice over controls (%ILS). An ILS of ≧ 25% represents activity. The tumor line was maintained through weekly transfer of 5 x $10^4$ cells in DBA/2 mice.

## Sarcoma 180 Ascites Screening Methodology

Female CFW mice (18 - 25 g) were implanted with 2 x $10^6$ cells ip on day zero. Compounds were administered in 0.5 mL water ip on day one. Groups of 6 mice were used for each test dose as well as controls as defined above. The test was run for twice the MST of the control group, with survivors counted as dying on that day. An ILS of ≧ 50% indicates activity. The tumor line was maintained through weekly transfer of 4 x $10^6$ cells in CFW mice.

The results of the screening test are reported in Tables 3 and 4.

### Table 3

S180a Antitumor Screening Data for Pt Triamines Containing Saturated Amines

| Compound (vehicle) | Dose mg/kg | %ILS | Surv. | Positive %ILS | Control Surv. |
|---|---|---|---|---|---|
| cis-[Pt($NH_3$)$_2$(4-methylpiperidine)Cl]$NO_3$ (water) | 10 | 4 | 0/6 | 80 | 3/6 |
| | 20 | 13 | 0/6 | | |
| | 40 | 18 | 0/6 | | |
| | 80 | 76 | 1/6 | | |
| | 160 | 4 | 1/6 | | |
| | 320 | -80 | 0/6 | | |
| cis-[Pt($NH_3$)$_2$(cyclohexanemethyl-amine)Cl]$NO_3$ (water) | 10 | 15 | 0/6 | 65 | 1/6 |
| | 20 | 22 | 0/6 | | |
| | 40 | 19 | 1/6 | | |
| | 80 | 61 | 0/6 | | |
| | 160 | -78 | 0/6 | | |
| | 320 | -94 | 0/6 | | |
| cis-[Pt($NH_3$)$_2$(octylamine)Cl]$NO_3$ (water) | 10 | 16 | 0/6 | 65 | 1/6 |
| | 20 | 25 | 0/6 | | |
| | 40 | 22 | 0/6 | | |
| | 80 | -78 | 0/6 | | |
| | 160 | -78 | 0/6 | | |
| | 320 | -95 | 0/6 | | |

Table 4

L1210 Antitumor Screening Data for Pt Triamines Containing Saturated Amines

| Compound (vehicle) | Dose mg/kg | %ILS | Surv. | Positive %ILS | Control Surv. |
|---|---|---|---|---|---|
| cis-[Pt($NH_3$)$_2$(4-methylpiperidine)Cl]$NO_3$ (water) | 10 | 24 | 0/6 | 149 | 2/6 |
| | 20 | 17 | 0/6 | | |
| | 40 | 60 | 0/6 | | |
| | 80 | 57 | 0/6 | | |
| | 160 | -28 | 0/6 | | |
| | 320 | -68 | 0/6 | | |

## Claims

1. The compounds of the general formula (I):

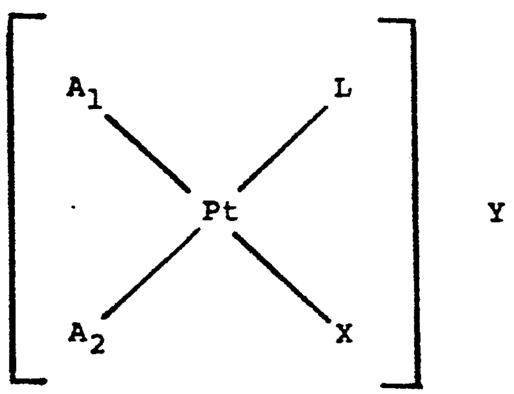

(I)

wherein Pt is in a valence state II and is coordinated to $A_1$ and $A_2$ in a cis configuration, wherein $A_1$ and $A_2$ are the same or different and are each selected from monodentate amines and substituted monodentate amines, or $A_1$ and $A_2$ together are a diamine represented by the general formula:

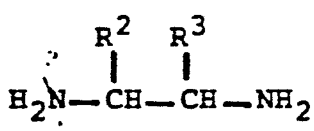

wherein $R^2$ and $R^3$ are the same or different and are each selected from linear or branched $C_1$-$C_6$ alkyl groups and substituted linear or branched $C_1$-$C_6$ alkyl groups, or $R^2$ and $R^3$ taken together, form a 1,2-diaminocycloalkane containing 4-8 nuclear carbon atoms or a substituted 1,2-diaminocycloalkane;
wherein L is different from $A_1$ and $A_2$ and is a $C_1$-$C_{12}$ linear, $C_1$-$C_{12}$ branched, $C_3$-$C_{12}$ cyclic, $C_5$-$C_{12}$ bicyclic, $C_2$-$C_{12}$ heterocyclic or $C_5$-$C_{12}$ bicyclic heterocyclic saturated amine or substituted $C_1$-$C_{12}$ linear, $C_1$-$C_{12}$ branched, $C_3$-$C_{12}$ cyclic, $C_5$-$C_{12}$ bicyclic, $C_2$-$C_{12}$ heterocyclic or $C_5$-$C_{12}$ bicyclic heterocyclic saturated amine;
wherein X is a monodentate anionic ligand; and wherein Y is an anion.

2. A compound according to claim 1, wherein X is monodentate anionic ligand selected from the group consisting of halo, hydroxo, pseudohalo, $HSO_4$, $H_2PO_4$, ascorbate and $C_1$-$C_6$ carboxylate; and wherein Y is halo, hydroxo, pseudohalo, $HSO_4$, $H_2PO_4$, ascorbate, $C_1$-$C_6$ carboxylate, nitrate or perchlorate.

3. A compound according to claim 1 or 2, wherein $A_1$ and $A_2$ are $NH_3$ or $R^1NH_2$, wherein $R^1$ is a substituted or unsubstituted, linear or branched $C_1$-$C_6$ alkyl group.

4. A compound according to claim 3, wherein the substituted linear or branched $C_1$-$C_6$ alkyl groups are substituted with one or more substituents selected from the group consisting of hydroxy, $C_1$-$C_4$ alkoxy, halo, carboxy, $C_1$-$C_4$ alkylcarboxy, $C_1$-$C_4$ alkoxycarbonyl and $C_3$-$C_6$ cycloalkyl.

5. A compound according to claim 1 or 2, wherein the substituted monodentate amine, the substituted 1,2-diaminocycloalkane and the substituted linear, branched, cyclic, bicyclic, heterocyclic or bicyclic heterocyclic saturated amines are substituted with one or more substituents selected from the group consisting of hydroxy, $C_1$-$C_4$ alkoxy, halo, carboxy, $C_1$-$C_4$ alkylcarboxy, $C_1$-$C_4$ alkoxycarbonyl and $C_3$-$C_6$ cycloalkyl and wherein the substituted cyclic, bicyclic, heterocyclic and bicyclic heterocyclic saturated amine may also or alternatively be substituted with a $C_1$-$C_4$ alkyl group.

6. A compound according to any of claims 1 to 5, wherein L is a $C_5$-$C_{12}$ bicyclic heterocylic saturated amine, a $C_3$-$C_{12}$ cyclic saturated amine, a $C_5$-$C_{12}$ bicyclic saturated amine, or a $C_2$-$C_{12}$ heterocyclic saturated amine.

7. A compound according to claim 1 that is selected from
cis-[Pt$(NH_3)_2$(4-methylpiperidine)Cl]$NO_3$,
cis-|Pt$(NH_3)_2$(cyclohexanemethylamine)Cl]$NO_3$,
cis-[Pt$(NH_3)_2$(isopropylamine)Cl]$NO_3$,
cis-[Pt$(NH_3)_2$(quinuclidine)Cl]$NO_3$ and
cis-[Pt$(NH_3)_2$(2-amino-1-methoxypropane)Cl]$NO_3$.

8. A composition for treating malignant animal tumours sensitive thereto comprising an active ingredient in admixture with a non-toxic pharmacologically acceptable inert carrier or diluent, characterised in that the active ingredient comprises a compound according to any one of claims 1 to 7.

9. A composition according to claim 8 in a form suitable for parenteral administration.

10. A composition according to claim B in a form suitable for oral administration.

11. A composition according to claim 8 in tablet form.

12. A composition according to claim 8 in capsule form.

13. A composition according to any of claims 8 to 12, containing the active ingredient in an amount sufficient to cause regression of the tumour cells.

14. A method of making a composition according to any one of claims 8 to 13, comprising mixing the said carrier or diluent with the active ingredient.

15. A method of treating malignant animal tumours sensitive to a compound according to any one of claims 1 to 7, comprising administering the compound or a mixture of such compounds to an animal afflicted with such a tumour in an amount sufficient to cause regression of the tumour cells.

16. A compound according to any of claims1 to 7, for use in the treatment of the human or animal body by therapy.

17. The use of a compound according to any of claims 1 to 7 in the manufacture of a medicament for treating animal malignant tumour cells.

18. A method of making a compound according to any of claims 1 to 7, wherein a compound of the general formula
$PtA_1A_2XY$
is reacted with a compound of the general formula L, in which Pt is in a valence state of II and is coordinated to $A_1$ and $A_2$ in a cis configuration and in which $A_1$, $A_2$, X, Y and L are as defined in claim 1.

19. A method according to claim 18, wherein the compound of the general formula
$PtA_1A_2XY$
is prepared by reacting a compound of the general formula
$PtA_1A_2X_2$
with a silver salt of the general formula AgY.